Europäisches Patentamt

European Patent Office

Office européen des brevets

(19)

(11) **EP 1 135 103 B1**

(12) **EUROPÄISCHE PATENTSCHRIFT**

(45) Veröffentlichungstag und Bekanntmachung des Hinweises auf die Patenterteilung:
**15.10.2003 Patentblatt 2003/42**

(21) Anmeldenummer: **99963345.6**

(22) Anmeldetag: **30.11.1999**

(51) Int Cl.⁷: **A61K 7/48**, A61K 7/06, A61K 35/78

(86) Internationale Anmeldenummer:
**PCT/EP99/09294**

(87) Internationale Veröffentlichungsnummer:
**WO 00/032162 (08.06.2000 Gazette 2000/23)**

(54) **VERWENDUNG VON PFLANZENEXTRAKTEN MIT ANTI-RADIKALARTIGER AKTION UND KOSMETISCHE ODER DERMOPHARMAZEUTISCHE ZUSAMMENSETZUNG, DIE DERARTIGE EXTRAKTE ENTHÄLT**

USE OF PLANT EXTRACTS WITH AN ANTI-RADICAL-TYPE ACTION AND COSMETIC OR DERMOPHARMACEUTICAL COMPOSITION COMPRISING SUCH EXTRACTS

UTILISATION D'EXTRAITS DE PLANTES AYANT NOTAMMENT UNE ACTION ANTI-RADICALE ET COMPOSITION COSMETIQUE OU DERMOPHARMACEUTIQUE COMPRENANT CES EXTRAITS

(84) Benannte Vertragsstaaten:
**AT BE CH CY DE DK ES FI FR GB GR IE IT LI LU MC NL PT SE**

(30) Priorität: **03.12.1998 FR 9815380**

(43) Veröffentlichungstag der Anmeldung:
**26.09.2001 Patentblatt 2001/39**

(73) Patentinhaber: **Cognis France S.A.**
**31360 Saint-Martory (FR)**

(72) Erfinder:
• **PAULY, Gilles**
**F-54000 Nancy (FR)**
• **MORETTI, Christian**
**F-75010 Paris (FR)**

(74) Vertreter: **Nuss, Pierre et al**
**Cabinet Nuss**
**10, rue Jacques Kablé**
**67080 Strasbourg Cédex (FR)**

(56) Entgegenhaltungen:
• **DATABASE WPI Week 9831 Derwent Publications Ltd., London, GB; AN 98-357423 XP002115098 & JP 10 139680 A (LION CORP.)**
• **STN, Serveur de Bases de Données, Karlsruhe, DE, Fichier Chemical Abstracts, Vol 93, AN=197959 * résumé * XP002115094**
• **STN, Serveur de Bases de Données, Karlsruhe, DE, Fichier Biosis, AN=1993:417511 * résumé * XP002115095**
• **STN, Serveur de Bases de Données, Karlsruhe, DE, Fichier Biosis, AN=1991:247007 * résumé * XP002115096**
• **STN, Serveur de Bases de Données, Karlsruhe, DE, Fichier Chemical Abstracts, Vol 66, AN=79488 * résumé * XP002115097**

**Beschreibung**

Die vorliegende Erfindung bezieht sich auf das Gebiet der Kosmetik, besonders auf die Verwendung von Pflanzenextrakten in der Dermo-Kosmetik und ihre Zielsetzung ist die Verwendung gewisser Heilpflanzen aus Französisch-Guayana für die Präparation kosmetischer oder dermopharmazeutischer Erzeugnisse für die Haut, die Schleimhäute und/oder die Epithel- oder Körperanhanggebilde (Haare, Nägel, ...).

Die kreolischen und palikurischen Völker aus Französisch-Guayana benutzen zahlreiche lokale Pflanzen in der herkömmlichen Medizin. Diese Pflanzen und ihr therapeutischer Gebrauch werden besonders im Werk: "Pharmacopées traditionnelles en Guyane: Créoles, Palikur, Wayapi" Grenand P., Moretti C, Jacquemin H., Edition de l'Orstom, 1987 beschrieben.

Weiterhin beschreibt DATABASE WPI, Week 9831, Derwent Publications Ltd., London, GB, AN 98-357423, XP002115098, wässrige oder organische Extrakte von Pflanzen, die u. a. Inga tete und Inga parensis enthalten. Besagte Extrakte werden zur Herstellung oraler Zusammensetzungen zur Behandlung von Kopfschuppen, Juckreiz, Haarausfall und Prostatahypertrophie verwendet.

Zudem offenbart STN, Serveur de Bases de Données, Karlsruhe, DE, Fichier Chemical Abstracts, AN = 1991 : 247007, XP002115096, u.a. wässrige Extrakte von byrsonima crassifolia zur Inhibierung von Dermatophyten.

Schließlich beschreibt STN, Serveur de Bases de Données, Karlsruhe, DE, Fichier Chemical Abstracts, vol. 93, AN = 197959, XP002115094, die Verwendung alkoholischer Extrakte, u.a. von Byrsonima verbascifolia, zur Aktivierung der Phagozyten im reticuloendothelialen System.

Aber die Autoren der vorliegenden Erfindung haben entdeckt, dass die Extrakte gewisser Pflanzen des vorab erwähnten Typs auch verschiedene biologische und biochemische Aktivitäten aufweisen, die mit einer sehr grossen kutanen Toleranz verbunden sind, wodurch sie direkt in Zusammensetzungen für den kosmetischen Gebrauch, besonders in der Dermo-Kosmetik, verwendbar gemacht werden.

Daher ermöglichen die von den Erfindern ausgewählten Pflanzen Extrakte zu gewinnen, die alle auf unerwartete und erstaunliche Weise eine verstärkte, anti-radikalartige Aktivität, aber auch hemmende Aktivitäten der Tyrosinase (entpigmentierend), Aktivierer der Tyrosinase (pigmentierend), Anti-UVA und Anti-UVB-Aktivitäten , sowie einen Schutz der Katalase gegen UVA, Anti-Elastase-, Anti-Kollagenase-, Anti-Glykation-und/oder lypolytische Aktivitäten (zum Schlank-werden) aufweisen.

Das Hauptziel der vorliegenden Erfindung besteht daher in seiner Verwendung als aktiver Wirkstoff, der besonders anti-radikalartige Aktivitäten aufweist, zur Präparation eines kosmetischen oder dermo-pharmazeutischen Erzeugnisses, für externe, lokale Anwendung auf der Haut, den Schleimhäuten und/oder dem Epitheloder Körperanhanggebilde von mindestens einem Extrakt einer Pflanze deren botanische Gattung zu den von den Clidemia, Inga, Sabicea, Astrocaryum, Siparuna, Eperua, Bysonima, Priva, Coutoubea und Goupia gebildeten Gruppe gehört.

In den vorab erwähnten Gattungen können folgende besondere Spezies vorteilhaft ausgewählt werden.

- Clidemia hirta, Clidemia dentata,
- Inga bourgoni, Inga pezizifera, Inga alata, Inga alba, Inga capitata, Inga meissneriana,
- Sabicea cinerea, Sabicea glabrescens, Sabicea velutina, Sabicea villosa, Sabicea hirsuta
- Eperua falcata,
- Byrsonima verbascifolia, Byrsonima crassifolia, Byrsonima chrysophylla, Byrsonima coriacea,
- Astrocaryum vulgare, Astrocaryum murumuru, Astrocaryum chambira, Astrocaryum jauari, Astrocaryum macrocalyx,
- Priva lappulacea,
- Coutoubea spicata, Coutoubea ramosa,
- Siparuna guianensis, Siparuna emarginata,
- Goupia glabra.

Aber nach einer bevorzugten Ausführung der vorliegenden Erfindung, die zu Extrakten führt, deren Aktivitäten qualitativ und quantitativ optimiert sind, besonders hinsichtlich der anti-radikalartigen Aktivität, besteht der aktive Wirkstoff aus mindestenes einem Extrakt einer Pflanze, die aus der durch Clidemia hirta, Inga bourgoni, Sabicea cinerea, Astrocaryum vulgare, Siparuna guianensis, Eperua falcata, Byrsonima verbascifolia, Priva lappulacea, Coutoubea spicata und Goupia glabra gebildeten Gruppe gewählt wurde.

Wegen ihrer zahlreichen Aktivitäten werden diese Extrakte auf vorteilhafte Weise allein oder untereinander verbunden oder mit anderen aktiven Verbindungen in Erzeugnissen verwendet, die hauptsächlich gegen die Bekämpfung kutaner Alterserscheinungen, kutane Hyperpigmentationen, Pigmentflecken, Elastizitätsverlust der Haut, Falten, Reizungen und Entzündungen (Behandlung sensibler Hauttypen), Umweltverschmutzung und/oder durch Sonne verursachte Reizerscheinungen und/oder für Schlankheitsmitteln bestimmt sind.

Die Teile der Pflanzen, die für die Präparation und die Gewinnung der vorab erwähnten Extrakte verwendet werden, werden unter den Wurzeln, den Rinden (die Wurzeln, die Stengel und/oder der Stamm der Pflanzen), den Blättern und beblätterten Stengeln, den Früchten, den Körnern und/oder den Blüten ausgewählt.

Nach dem Pflücken werden die Teile der entsprechenden Pflanzen nach dem Trocknen einem Extraktionsverfahren unterzogen, das verwendete Lösemittel kann auf vorteilhafte Weise in der durch Wasser, Akohole, Ketone, Ester, Äthertypen, chlorhaltige Lösemittel, Polyole gebildeten Gruppe oder durch Mischungen von mindestens zwei der vorab erwähnten mischbaren Lösemittel gewählt werden.

Nach einer besonderen Ausführung der vorliegenden Erfindung wird der Extrakt oder werden die Extrakte mittels eines Extraktionsverfahrens gewonnen, der auf einer Wellenausstrahlung, wie zum Beispiel Mikrowellen oder Ultraschall beruht.

Die Extrakte, von der die vorliegende Erfindung handelt, können auch durch die Extraktion von superkritischem $CO_2$, allein oder in einer Mischung mit einem Beilösemittel gewonnen werden.

Nach einer besonderen Ausführung der vorliegenden Erfindung besteht/bestehen der/die Extrakt/e der Pflanzen aus einer oder mehreren isolierten, besonders durch Chromatographie gereinigten Fraktion/en, ausgehend von einem oder mehreren Extrakt/en der besagten Pflanzen, der/die durch eines der vorab erwähnten Extraktionsverfahren gewonnen wurde/n.

Zur Veranschaulichung, aber nicht zur Einschränkung werden nachstehend verschiedene Verfahren beschrieben, die für die Ausführung der erwähnten Pflanzenextrakte vorgenommen werden können.

Die verwendeten Teile der Pflanze werden in den nachstehenden Beispielen nur als Angabe genannt, die Extrakte, von denen die vorliegende Erfindung handelt, können aus allen zugänglichen Teilen der vorab angegebenen Pflanzen gewonnen werden.

Beispiel 1

Rinden des Epurea falcata-Stamms werden grob zerkleinert und dann fein durch den Messerquetscher zerquetscht.

In einen mit einem Rührer versehenen Reaktor werden drei Liter destilliertes Wasser eingeleitet und dann werden nacheinander die folgenden Vorgänge vorgenommen, die bestehen aus:

- dem Hinzufügen von 300 g zerquetschter Rinde in den Reaktor,
- Extraktion unter Rühren während einer Stunde bei Sieden,
- Abkühlen auf Raumtemperatur,
- Beseitigung der unlösbaren Stoffe durch Zentrifugieren oder Filtern,
- Filtern der an der Oberfläche schwimmenden Stoffe bis auf eine Porosität von ungefähr 0,45 $\mu$m,
- Auffangen des Filtrats,
- Extraktion des Restes unter den gleichen Betriebsbedingungen durch 2 Liter destilliertes Wasser und wie vorab erwähnt vorgehen,
- nach der Bestimmung des Gehalts an trockener Materie, im Verhältnis zu den gewonnenen wässrigen Extrakten einen Zusatz des Typs Maltodextrin hinzufügen, um 2/3 Zusatz für 1/3 extrahierter, trockener Materie zu gewinnen,
- die gewonnene Lösung durch Zerstäubung oder durch eine andere, dem Fachmann auf dem Gebiet bekannte Technik entwässern (deshydratisieren Gefriertrocknung - Lyophilisierung, Trocknen im Trockenschrank usw.)

Der Gesamtertrag an trockenem Extrakt dieses Verfahrens beträgt 15 % im Gewicht im Verhältnis zu den Rinden.

Beispiel 2

Rinden des Inga bourgoni-Stamms werden grob zerkleinert und dann fein mit dem Messerquetscher zerquetscht.

In einen mit einem Rührer versehenen Reaktor werden drei Liter 50 %iger Äthylalkohol (Äthanol) eingeleitet und dann werden nacheinander die folgenden Vorgänge vorgenommen, die bestehen aus:

- dem Hinzufügen von 300 g zerkleinerter Rinde in den Reaktor,
- Extrahieren unter Rühren während einer Stunde unter Erhitzen (unter Rückfluss),
- Abkühlen auf Raumtemperatur,
- Beseitigung der unlösbaren Stoffe durch Filtern,
- Filtern der auf der Oberfläche schwimmenden Stoffe bis auf eine Porosität von ungefähr 0,45 $\mu m$,
- Auffangen des Filtrats,
- Extrahieren des Restes unter den gleichen Betriebsbedingungen durch 1,5 Liter 50 %igen Äthanol und dann wie oben beschrieben vorgehen,

- die Alkoholphase der beiden Filtrate unter Vakuum verdampfen lassen,
- nach dem Zentrifugieren und der Bestimmung des Gehalts an trockener Materie, falls erforderlich den Zusatz zu den gewonnenen wässrigen Phasen in den gleichen Verhältnissen wie die im Beispiel 1 erwähnten, hinzufügen,
- falls erforderlich die Lösung nach den herkömmlichen Techniken entwässern (deshydratisieren).

Der Gesamtertrag an trockenem Extrakt dieses Verfahrens beträgt 13,8 % im Gewicht im Verhältnis zu den Rinden.

Beispiel 3

Rinden der Wurzeln des Byrsonima verbascifolia werden grob zerkleinert und dann fein durch den Messerquetscher zerquetscht.

In einen mit einem Rührer versehenen Reaktor werden 3 Liter 80 %iger Methylalkohol (Methanol) eingeleitet und dann werden nacheinander die folgenden Vorgänge vorgenommen, die bestehen aus:

- dem Hinzufügen von 300 g zerkleinerter Rinde in den Reaktor,
- dem Extrahieren unter Rühren während einer Stunde unter Erhitzen (unter Rückfluss),
- Abkühlen auf Raumtemperatur,
- Beseitigung der unlösbaren Stoffe durch Filtern,
- Filtern der auf der Oberfläche schwimmenden Stoffe bis auf eine Porosität von ungefähr 0,45 $\mu$m,
- Auffangen des Filtrats,
- Extrahieren des Restes unter den gleichen Betriebsbedingungen durch 1,5 Liter 80 %igen Methylalkohol und danach wie vorab beschrieben vorgehen,
- unter Vakuum die Methanolphase der beiden Filtrate verdampfen lassen,
- nach dem Zentrifugieren und der Bestimmung des Gehalts an trockener Materie den Zusatz zur gewonnenen wässrigen Phase in den gleichen Proportionen wie die im Beispiel 1 beschriebenen hinzufügen,
- falls notwendig, die Lösung nach den herkömmlichen Techniken entwässern.

Der Gesamtertrag an nach diesem Verfahren gewonnenem Extrakt beträgt 17,4 % im Gewicht im Verhältnis zu den Rinden.

Beispiel 4

Wurzeln von Astrocaryum vulgare werden grob zerkleinert und dann fein zerquetscht.

In einen mit einem Rührer versehenen Reaktor werden 3 Liter absolutes Äthanol eingeleitet und dann werden nacheinander die folgenden Vorgänge vorgenommen, die bestehen aus:

- dem Hinzufügen von 300 g grob zerkleinerten Astrocaruym vulgare Wurzeln in den Reaktor,
- Extrahieren unter Rühren während einer Stunde unter Erhitzung (unter Rückfluss),
- Abkühlen auf Raumtemperatur,
- Beseitigung der unlösbaren Stoffe durch Filtern,
- Filtern der an der Oberfläche schwimmenden Stoffe bis auf eine Porosität von ungefähr 0,45 $\mu$m,
- Auffangen des dem Extrakt 1 entsprechenden Filtrats,
- Extrahieren des Rests unter den gleichen Betriebsbedingungen durch 3 Liter Äthanol und dann wie vorab beschrieben vorgehen, um den Extrakt 2 zu gewinnen,
- die Äthanhische Phase der beiden Filtrate unter Vakuum bei 40° C verdampfen lassen,
- Beseitigung der Spuren des Lösemittels durch das Trocknen der Extrakte in einem belüfteten Trockenofen bei 40-50° C.

Auf diese Weise werden 7,15 g E1-Extrakt und 1,47 g E2-Extrakt gewonnen, die zum Schluss vermischt werden, was einem Gesamtertrag an Extrakt von 2,87 % im Gewicht im Verhältnis zu den Wurzeln entspricht.

Die folgende Tabelle bietet als Angabe eine Aufstellung aller Pflanzen und Extrakte, mit denen die Erfinder gearbeitet haben.

| Pflanze | Extrahierte Pflanzenteile | Extrakttyp | Ertrag % (2 aufeinander-Folgende Extraktionen) | Aspekt |
|---|---|---|---|---|
| Clidemia hirta | Blätter | wässriger Extrakt | 20,9 | braunes Pulver |
| Inga bourgonl | Rinden | wässriger Extrakt | 9,7 | weinrotes Pulver |
|  |  | Ätnanot-E. 50 % | 13,8 | weinrotes Pulver |
|  |  | Äthanol-Extrakt | 11,7 | weinrotes Pulver |
| Sabicea cinerea | beblätterte Stengel | wässriger Extrakt | 16,4 | braunes Pulver |
|  |  | Äthanol-E. 50 % | 25,5 | braunes Pulver |
|  |  | Äthanol-Extrakt | 18,7 | grüne Paste |
| Astrocaryum vulgare | Wurzeln | wässriger Extrakt | 5,3 | braunes Pulver |
|  |  | Äthanol-E. 50 % | 5,6 | braunes Pulver |
|  |  | Äthanol-Extrakt | 2,8 | braun-orange Paste |
| Siparuna guianensis | Blätter | wässriger Extrakt | 16,1 | braunes Pulver |
|  |  | Methanol-E. 80 % | 21,9 | braunes Pulver |
|  |  | Äthanol-Extrakt | 22,6 | grüne Paste |
| Eperua falcata | Rinden | wässriger Extrakt | 15 | braunes Pulver |
|  |  | Methanol-E. 80 % | 17,6 | braunes Pulver |
|  |  | Äthanol-Extrakt | 16,7 | braune Paste |
| Byrsonima verbascifolia | Rinden der Wurzeln | wässriger Extrakt | 10,8 | braunes Pulver |
|  |  | Methanol-E. 80 % | 17,4 | braunes Pulver |
|  |  | Äthanol-Extrakt | 15,7 | braune Paste |
| Priva lappulacea | beblätterte Stengel | wässriger Extrakt | 24,0 | braunes Pulver |
|  |  | Methanol-E. 80 % | 14,5 | braunes Pulver |
|  |  | Äthanol-Extrakt | 13,9 | braune Paste |
| Goupia glabra | Blätter | wässriger Extrakt | 30,0 | braun-orange Pulver |
|  |  | Methanol-E. 80 % | 30,4 | gelbliches Pulver |
| Coutoubea spicata | Stengel+Ähren | wässriger Extrakt | 18,5 | braunes Pulver |
|  |  | Methanol-E. 80 % | 18,8 | braunes Pulver |
|  |  | Äthanol-Extrakt | 18,0 | braun-grüne Paste |

Die biologischen und biochemischen Eigenschaften und Aktivitäten der studierten Pflanzenextrakte, die direkt in den in kosmetischer Hinsicht interessanten Erzeugnissen verwendbar sind, konnten durch Tests bestimmt und gemessen werden, die dem Fachmann auf diesem Gebiet bekannt sind und deren Grundsätze nachstehend kurz erörtert werden und deren Ergebnisse in den entsprechenden Tabellen aufgestellt sind.

**I) Anti-radikalartige Aktivitäten**

Die oxidativen Anti-Stress-Fähigkeiten sind durch "in tubo"- und "in vitro"-Tests ausgewertet worden.

Die Gruppe der in tubo-Tests umfasst sowohl die anfänglichen radikalartigen Formen, als auch die in vivo eingeleiteten reaktiven Formen des Sauerstoffs: radikales Hydroxyl ($HO^\circ$ und Anion Superoxyd ($O_2^{\overline{\circ}}$).

### 1) "Chemie"-Tests in tubo

a) Anti-DPPH-Test

DPPH (Diphenylpikryl-Hydrazyl) ist ein freies, beständiges und violett gefärbtes Radikal, das in seinem Leukoderivat durch die Substanzen verändert wird, die die freien Radikale einfangen (neutralisierender Effekt, auch als "Scavenger-Effekt" bezeichnet).
Das Ergebnis wird in prozentualer Hemmung des DPPH° in radikalartiger Form im Verhältnis zum Kontrollmittel ohne Extrakt angegeben.

b) Anti-HO°-Test mit Salizylsäure (Fenton-Reaktion)

Die HO° (durch $H_2O_2$ bei Vorhandensein von $Fe^{++}$ und EDTA gebildet) hydroxylieren die Salizylsäure, die dann eine rötliche Verbindung bildet.
Die optische Dichte bei 490 nm entspricht dem hydroxylierten Salizylsäuregehalt
Eine anti-radikalartige Substanz reagiert mit den HO°-Radikalen und reduziert die Bildung dieser rot gefärbten Verbindung.
Die Ergebnisse werden in prozentualer Hemmung des Hydroxylationsgehalts (im Durchschnitt auf 2 Versuche) angegeben.

c) Anti-HO°-Test mit Desoxyribose (Fenton-Reaktion)

Die HO° (durch $H_2O_2$ bei Vorhandensein von $Fe^{++}$ und EDTA gebildet) oxydieren die Desoxyribose in aldehydische Derivate, die dann durch Thiobarbituratsäure dosiert werden.
Die Thiobarbituratsäure bildet durch Kondensation mit den Aldehyden eine rosa Verbindung (DO bei 532 nm).
Diese Fenton-Reaktion wird auch ohne EDTA realisiert, um die Fähigkeiten Eisen zu komplexieren, zu messen, wobei die Desoxyribose durch HO° oxydiert wird.
Die Ergebnisse werden in prozentualer Hemmung (im Durchschnitt auf 2 Versuche) angegeben.

### 2) "Biochemie" in tubo-Tests = Anti-Anion-Superoxyd $O_2^{-\circ}$-Tests

Die Xanthin-Oxydase ist ein während des oxydativen Stresses eingeführtes Enzym, das die Purinbasen (Adenin, Guanin) in Harnsäure und $O_2^{-\circ}$ katabolisiert.
$O_2^{-\circ}$ dismutiert sich spontan (oder durch SOD = Superoxyd-Dismutase) in $H_2O_2$ und $O_2$.

a) Aktivität Anti-$O_2^{-\circ}$: Methode mit Luminol.

$O_2^{-\circ}$ kann hinsichtlich der Lumineszenz durch Luminol erkannt werden.
Ein enzymatisches System (Hypoxanthin/Xanthin-Oxydase) bildet die Superoxyd-Anione $O_2^{-\circ}$, die mit Luminol reagieren, um eine Zwischenverbindung zu bilden, die, wenn sie sich stabilisiert eine Lumineszenz abgibt, die mittels eines Photovervielfachers (Photomultiplikators) aufgenommen wird.
Eine Substanz mit anti-radikalartiger Aktivität absorbiert oder zerstört das Anion $O_2^{-\circ}$ und reduziert dadurch die Bildung von Lumineszenz.

b) Anti-$O_2^{-\circ}$ und $H_2O_2$-Aktivität: Methode mit Luminol bei Vorhandensein von Mikroperoxydase

Ein enzymatisches System (Hypoxanthin/Xanthin-Oxydase) bildet die Superoxyd-Anione $O_2^{-\circ}$, die sich langsam in $O_2^{-\circ}$ und $H_2O_2$ dismutieren.
$H_2O_2$ und $O_2^{-\circ}$ reagieren mit der Mikroperoxydase (M), um $O_2^1$ (Singulett-Sauerstoff) zu bilden, das Luminol (Lum) degradiert, um eine lumineszierende Verbindung zu bilden, deren Lichtabgabe mittels eines Photovervielfachers aufgenommen wird.
Eine anti-radikalartige Substanz absorbiert entweder $H_2O_2$ oder $O_2^{-\circ}$ oder $O_2^1$ und reduziert dadurch die Bildung von Lumineszenz.

c) Anti-$O_2^{-\circ}$-Aktivität: Methode mit NBT (Tetrazoliumsalz)

Ein enzymatisches System (Hypoxanthin/Xanthin-Oxydase) bildet Superoxyd-Anione $O_2^{-\circ}$, die mit dem NBT reagieren, um eine rote Verbindung zu bilden: das Formazan.

Eine anti-radikalartige Substanz absorbiert oder zerstört $O_2^{\overline{\circ}}$ und reduziert dadurch die Bildung von Formazan.

Die Ergebnisse dieser drei Tests a), b) und c) werden in prozentualer Hemmung angegeben (im Durchschnitt auf 2 Versuche).

Die Ergebnisse der oben erwähnten Tests 1), und 2) werden in folgender Tabelle aufgestellt:

| Pflanze | Extrakttyp | ARL (% I) | Fenton-Test (% I) | HX + XOD-Test (% I) | | | |
|---|---|---|---|---|---|---|---|
| | | DPPH | Salizylsäure | Desoxyribose | Lum | Lum+M | NTB |
| Clidemia hirta | wässriger Extrakt | 91 | 40 | 70 | 99 | 51 | 53 |
| Inga bourgoni | wässriger Extrakt | 94 | | 40 | 100 | 98 | 64 |
| | Äthanol-E. 50 % | 94 | | 27 | 100 | 98 | 71 |
| | Äthanol-Extrakt | 95 | | 74 | 100 | 99 | 83 |
| Sabicea cinerea | wässriger Extrakt | 90 | 47 | 39 | 99 | 89 | 41 |
| | Äthanol-E. 50 % | 94 | 39 | 50 | 100 | 99 | 62 |
| | Äthanol-Extrakt | 93 | 51 | | 100 | 98 | 50 |
| Astrocaryum vulgare | wässriger Extrakt | 76 | | | 98 | 27 | 23 |
| | Äthanol-E. 50 % | 93 | | | 100 | 98 | 74 |
| | Äthanol-Extrakt | 93 | 48 | 59 | 100 | 100 | 81 |
| Siparuna guianensis | wässriger Extrakt | 92 | | | 99 | 67 | 47 |
| | Methanol-E. 80 % | 94 | | 47 | 100 | 99 | 71 |
| | Äthanol-Extrakt | 38 | 49 | | 99 | 96 | 54 |
| Eperua falcata | wässriger Extrakt | 91 | | | 100 | 85 | 49 |
| | Methanol-E. 80 % | 91 | | | 99 | 94 | 61 |
| | Äthanol-Extrakt | 94 | | 64 | 100 | 99 | 89 |
| Byrsonima verbascifolia | wässriger Extrakt | 95 | | 80 | 100 | 100 | 83 |
| | Methanol-E. 80 % | 95 | | 84 | 100 | 100 | 75 |
| | Äthanol-Extrakt | 94 | | 75 | 100 | 100 | 80 |
| Priva lappulacea | wässriger Extrakt | 95 | 46 | | 100 | 70 | 35 |
| | Methanol-E.80 % | 100 | 51 | 45 | 100 | 97 | 45 |
| | Äthanol-Extrakt | 93 | 37 | | 100 | 96 | 44 |

(fortgesetzt)

| Pflanze | Extrakttyp | ARL (% I) | Fenton-Test (% I) | HX + XOD-Test (% I) | | |
|---|---|---|---|---|---|---|
| | | DPPH | Salizylsäure | Desoxyribose | Lum | Lum+M | NTB |
| Goupia glabra | wässriger Extrakt | 96 | 49 | 32 | 100 | 94 | 34 |
| | Methanol-E. 80 % | 89 | 61 | 31 | 100 | 90 | |
| Coutoubea spicata | wässriger Extrakt | 92 | 79 | | 99 | 41 | |
| | Methanol-E. 80 % | 93 | 81 | | 100 | 89 | |
| | Äthanol-Extrakt | 92 | 72 | | 99 | 50 | |
| Lum: Luminol; Lum + M: Luminol + Mikroperoxydase | | | | | | | |

## II) Anti-UVA-Cytoschutz auf menschlichen Fibroblasten in "in vitro"-Überleben

A) Dosierung des ausgesalzenen MDA (Morlière P. u. Koll.: "UV-A induced lipid peroxydation in cultured human fibroblasts", Biochim. Biophys. Acta 1084, 3 : 261-269, 1991).

### 1) Prinzip

Die UV-A dringen bis in die Lederhaut ein, wo sie einen oxydativen Stress einleiten, der besonders durch eine Lipoperoxydation der zytoplasmischen Membrane angezeigt wird.

Die Lipoperoxyde fragmentieren sich in Malonaldialdehyd, das zahlreiche biologische Moleküle, wie zum Beispiel Proteine (Enzymhemmung) und die Nukleinbasen (Mutagenese) vernetzen/verbinden wird.

### 2) Ausführung

Die Fibroblaste werden in einen Nährboden geimpft, der mit Kalbsfötenserum definiert ist.

Der Pflanzenextrakt (im mit 2 % Serum definierten Nährboden) wird 72 Stunden nach dem Einimpfen hinzugefügt.

Nach einer 48-stündigen Inkubation bei 37° C und $CO_2$ = 5 % wird der Nährboden durch eine Salzlösung ersetzt und die Fibroblaste werden mit einer UV-A-Dosis (15 J/cm$^2$) bestrahlt.

Nach dem Ende der Bestrahlung wird die MDA-Rate (Malonaldialdehyd) in der auf der Oberfläche schwimmenden Salzlösung dosiert und die Proteinrate wird in den Fibroblasten gemessen. MDA wird durch die Reaktion auf Thiobarbituratsäure dosiert und die Proteine nach der sogenannten Bradford-Methode.

### B) Schutz der Aktivität des Katalaseenzyms gegen UVA

### 1) Prinzip

UVA dringen bis zur Lederhaut ein, wo sie einen oxydativen Stress einleiten, der eine Senkung der Aktivität zahlreicher Enzyme, unter anderem der Katalase verursacht.

Die Katalase ist ein Enzym, das die Beseitigung des spontan durch die Oxydierung von Substraten, wie zum Beispiel der Glukose oder dem Hypoxanthin erzeugten $H_2O_2$ ermöglicht.

$H_2O_2$ muss schnell beseitigt werden, weil er sonst beim Vorhandensein von Eisen sehr giftige (toxische) Hydroxylradikale (HO°) bildet.

### 2) Ausführung

(von LH. Johansson und LAH. Borg in "A spectrophometric method for determination of catalase activity in small tissue samples", Anal. Biochem. 174, 331-336, 1988).

Fibroblaste werden in einen durch Kalbsfötenserum definierten Nährboden geimpft.

Der Pflanzenextrakt (in der Konzentration von 0,01 bis 0,05 % Gew./V in einem definierten Nährboden aufgelöst, der 2 % Serum enthält), wird 72 Stunden nach dem Einimpfen hinzugefügt.

Nach einer 48-stündigen Inkubation bei 37° C und $CO_2$ = 5 % wird der Nährboden durch eine Salzlösung ersetzt und die Fibroblaste werden mit einer UVA-Dosis (5 $J/cm^2$) bestrahlt.

Nach dem Ende der Bestrahlung wird die Rate der aktiven Katalase nach einer spektrophotometrischen Methode dosiert und ihre Aktivität wird auf die in den Fibroblasten gemessene Proteinrate bezogen.

Die Wirkung der Pflanzenextrakte wird in prozentualer Steigerung der Katalaseaktivität im Verhältnis zu den bestrahlten Kontrolimitteln in Abwesenheit von Pflanzenextrakt angegeben (im Durchschnitt zwei Versuche).

**III) Nachweis des Anti-UVB-Cytoschutzeffekts auf menschlichen Keratinozyten im "in vitro"-Überleben**

**1) Prinzip**

UV-B lösen eine leichte Entzündung aus (Erythem, Ödem) durch die Aktivierung eines Enzyms, der Phospholipase A2.

Dieses Enzym entreisst den Phospholipiden der plasmischen Membran die Arachidonsäure: die Arachidonsäure ist der Vorläufer der Prostaglandine, darunter auch der E2-Prostaglandine (= PGE2), die durch Cyclooxygenase gebildet werden.

**2) Ausführung**

A431-Keratinozyte werden in einen mit Kalbsfötenserum definierten Nährboden geimpft. Nach einer 72stündigen Inkubation bei 37° C und $CO_2$ = 5 % wird der Nährboden durch eine Salzlösung ersetzt, die den zu testenden Pflanzenextrakt enthält. Die Keratinozyten werden sogleich mit einer UV-B-Dosis (30 $mJ/cm^2$) bestrahlt, 24 Stunden lang bei 37° C inkubiert, dann werden die PGE2- und LDH-Raten im an der Oberfläche schwimmenden Nährboden gemessen.

Die Anzahl der festhaftenden Keratinozyte wird durch einen Partikelzähler bestimmt (nach Trypsination) und die PGE2-Rate wird durch einen ELISA-Test bestimmt.

Durch eine enzymatische Reaktion wird die LDH-Rate (Laktatedeshydrogenase) ebenfalls bestimmt, die ein zytoplasmisches Enzym ist, das Zellenleiden markiert.

Die Aktivität des Pflanzenextrakts wird getestet und in prozentualer Hemmung der beiden Markierer im Verhältnis zu den mit den Kontrollmitteln erreichten Werten angegeben (im Durchschnitt 2 Versuche, jeder in doppelter Ausfertigung).

**IV) "Anti-Protease"-Aktivität**

Die Protease, die von den neutrophilen Polymorphonuklearen während einer leichten Entzündung oder durch einer UV-A-Bestrahfung ausgesetzten Fibroblaste, abgesondert wurden, rufen einen Abbau der Proteine hervor, die die extrazellulare Matrix der Lederhaut strukturieren.

Zwei Proteasetypen wurden durch enzymatische Reaktionen in tubo getestet.

**1) Anti-Elastase-Test** (Bieth J., "Elastase: Structure, Function and Pathological role", Front Matrix Biol. 6: 1-82, Karger, 1978)

Die PNN sondern eine auf Elastine aktive Elastase (Serin-Protease) ab, die Proteoglykane und die Kollagene.

Die in tubo-Tests wurden auf einer Elastase der Bauchspeicheldrüse (Serin-Protease) mittels zweier Substrattypen vorgenommen: einem synthetischen chromogenen (SS-Test) und einem natürlichen Substrat, das mit Rotkongo (Rc-Test) verbundenes Elastin ist.

Die Inkubationen dauern 30 Minuten bei Raumtemperatur oder 2 Stunden bei 37° C und die Färbungen werden jeweils bei 410 und bei 520 nm gemessen.

Das zum Vergleich getestete Hemmungsprüfmass ist das $\alpha$ 1 Antitrypsin.

**2) Anti-Kollagenase in tubo-Test** (Van Wart u. Koll., "A continuous spectrophotometric assay for Clostridium histolyticum collagenase", Anal. Biochem., 113, 356-365, 1981).

Die PNN während einer leichten Entzündung und die "alten" oder bestrahlten Fibroblaste sondern Kollagenasen ab.

Die Tests wurden mit einer Clostridium hystoliticum-Kollagenase und einem synthetischen chromogenen Substrat: dem FALGPA ausgeführt.

Die Inkubation dauert 30 Minuten bei Raumtemperatur und die optische Dichte wird bei 324 nm gemessen.

Die Ergebnisse werden in prozentualer Hemmung des Enzyms im Verhältnis zum Kontrollmittel ohne Aktiv angegeben.

Das zum Vergleich getestete Hemmungskontrollmittel ist Zystein.

Die Ergebnisse der Testserien **II), III) und IV)** werden in der folgenden Tabelle aufgeführt:

| Pflanze | Extrakttyp | UV-A (%I) | Katalase % Steigerung Aktivität/ bestrahltes Kontrollm. | UV-B (% I) | | Elastase (%1) | | Kollagenase (% I) |
|---|---|---|---|---|---|---|---|---|
| | | MDA | | PGE2 | LDH | Rc | SS | |
| Clidemia hirta | wässriger Extrakt | 23 | | | 67 | 92 | 67 | 93 |
| Inga bourgoni | wässriger Extrakt | 71 | | 78 | 92 | 72 | 34 | 100 |
| | Äthanol-E. 50 % | 70 | | 58 | 85 | 91 | 38 | 100 |
| | Äthanol-Extrakt | | | 100 | 100 | 61 | | |
| Sabicea cinerea | wässriger Extrakt | 74 | | 95 | 100 | 68 | | 83 |
| | Äthanol-E. 50 % | 86 | | 40 | 75 | 60 | | 100 |
| | Äthanol-Extrakt | 78 | 81 | 87 | 100 | 47 | | 27 |
| Astrocaryum vulgare | wässriger Extrakt | 91 | | 78 | 97 | 9 | | 15 |
| | Äthanol-E. 50 % | 82 | | 68 | 79 | 29 | 56 | 100 |
| | Äthanol-Extrakt | 75 | | 79 | 83 | 100 | 75 | 91 |
| Siparuna guianensis | wässriger Extrakt | 79 | | 60 | 86 | 29 | | 36 |
| | Methanol-E. 80 % | 72 | 55 | 50 | 75 | 78 | 62 | 99 |
| | Äthanol-Extrakt | | | | | | | 21 |
| Eperua falcata | wässriger Extrakt | 79 | | 100 | 100 | | | 36 |
| | Methanol-E. 80 % | 66 | | 99 | 100 | | | 85 |
| | Äthanol-Extrakt | 59 | | 88 | 93 | 49 | | 72 |

(fortgesetzt)

| Pflanze | Extrakttyp | UV-A (%I) | Katalase % Steigerung Aktivität/ bestrahltes Kontrollm. | UV-B (% I) | | Elastase (%1) | | Kollagenase (% I) |
|---|---|---|---|---|---|---|---|---|
| | | MDA | | PGE2 | LDH | Rc | SS | |
| Byrsonima verbascifolia | wässriger Extrakt | 63 | 23 | 70 | 92 | 100 | 66 | 100 |
| | Methanol-E. 80 % | 66 | 26 | 64 | 89 | 101 | 86 | 100 |
| | Äthanol-Extrakt | 42 | 24 | 50 | 73 | 100 | 68 | 100 |
| Priva lappulacea | wässriger Extrakt | 26 | | 21 | 27 | | | |
| | Methanol-E. 80 % | | | | | | | |
| | Äthanol-Extrakt | | | | | | 70 | 99 |
| Goupia glabra | wässriger Extrakt | | | | | | | |
| | Methanol-E. 80 % | | | | | | | |
| Coutoubea spicata | wässriger Extrakt | | | | | | | |
| | Methanol-E. 80 % | | | | | | | |
| | Äthanol-Extrakt | | | | | | | 47 |
| MDA: Malonaldialdehyd: PGE2: Prostaglandin E2; LDH: Laktat-Deshydrogenase; %: Hemmungsprozentsatz im Verhältnis zum Kontroll-mittel | | | | | | | | |

## V) Hemmung der Melanogenese

### 1) Hemmung der Tyrosinase "in tubo"

Die Tyrosinase ist das Schlüsselenzym der Synthese des Melanins in den Melanozyten der menschlichen Haut.

Dieses Enzym katalysiert die ersten beiden Etappen der Verwandlung des Tyrosins in Melanin, das heisst: die Oxydation des Melanins in DOPA (Dihydroxyphenylalanin) und danach in Dopachrom.

Dopachrom ist eine durch Spektrophotometrie bei 475 nm quantifizierte gefärbte Verbindung.

Die Aktivität des in tubo verwendeten Enzyms (aus Pilzen gewonnen) wird durch die Reaktionskinetik auf 20 Sekunden bestimmt.

Die Prüfmasssubstanz ist Hydroquinon (CI50 = 0,025 %, Gew./v)

Die Ergebnisse werden in prozentualer Hemmung im Verhältnis zum Kontrollmittel ausgedrückt.

### 2) Hemmung der Melanogenese auf B16-Melanozyten in der "in vitro"-Kultur

Die Hemmung der Melanogenese wird durch spektrophotometrische Dosierung des durch Melanozyten (B16-Linie) erzeugten Melanins ausgewertet, die "in vitro" bei Vorhandensein des zu testenden Pflanzenextrakts inkubiert wurden.

Die B16-Melanozyten werden in einem definierten Wachstumsboden kultiviert und 3 Tage lang bei 37° C, $CO_2$ = 5 % inkubiert.

Die Pflanzenextrakte werden in einem Medium aufgelöst, das die Melanogenese aktiviert und sie werden dann 3 Tage lang bei 37° C in Kontakt mit den B16 gebracht.

Die Rate des ausgesalzenen Melanins im Medium, das auf der Oberfläche schwimmt, wird durch Spektrophotometrie bei 475 nm quantifiziert.

Die Rate des intrazellularen Melanins wird durch Spektrophotometrie bei 475 nm im Homogenat der B16-Zellen quantifiziert, die in einer NaOH 1N + 10 % (v/v) DMSO-Mischung aufgelöst wurden.

Die Proteinrate im Homogenat der B16-Zellen wird nach der sogenannten Bradford-Methode quantifiziert.

Die Vergleichssubstanzen sind Hydroquinon und Arbutin.

## VI. Nachweis einer Hemmungsaktivität der nicht-enzymatischen in-tubo-Glykation ("Anti-Glykation"-Aktivität)

(DEYL und Koll. "Increased glycation and pigmentation of collagen in aged and young parabiotic rats and mice", Mechanisms of ageing and development, 55, 39-47, 1990 / MONNIER und Koll., "Accelerated age-related browning of human collagen in diabetes mellitus", Proc. Natl. Acad. Sci., USA: 81 583 - 587, 1984)

Die nicht-enzymatische Glykation der Proteine ist ein entscheidender Vorgang des Alterns der menschlichen Gewebe.

Diese von Maillard entdeckte Reaktion erklärt die Retikulation der extrazellularen Matrixen und der Basenmembranen, die zum grossen Teil für bei Diabetikern beobachteten Pathologien verantwortlich sind.

Ausserdem katalysieren die Schiff-Basen die Erzeugung reaktiver Formen des Sauerstoffs, die die Wirkungen der nicht enzymatischen Glykation verschlimmern.

Die in tubo-Tests werden auf Kollagen des I-Typs vorgenommen, das 21 Tage lang bei 45° C beim Vorhandensein von Glukose bei 1 % (p/v) inkubiert.

Die Rate der Schiff-Basen wird durch Fluorimetrie bei 430 nm (Erregung bei 350 nm) in den an der Oberfläche schwimmenden Stoffen (fs) und im Zentrifugations-Bodensatz (fgc) ausgewertet. Das Ergebnis wird in prozentualer Hemmung im Verhältnis zum Kontrollmittel ohne Pflanzenextrakt angegeben.

## VII) Aktivierung der Lipolyse auf menschlichen Adipozyten im "in vitro"-Überleben

Die Lipolyse ist die Beseitigung der Triglyzeride (oder TG), die in den Adipozyten durch ein Enzym, die Triglyzerid-Lipase (oder TGL) gespeichert sind, die die Spaltung der TG in freie Fettsäure und Glyzerin, die im Blutkreislauf eliminiert werden, hervorruft

Die Fettsäuren können dann von Muskelzellen aufgenommen werden, um Energie zu erzeugen.

Der in vitro ausgeführte Test erfolgt wie nachstehend beschrieben:

Die Aktivierung der Lipolyse wird durch spektrophotometrische Dosierung der Rate des durch Adipozyte ausgesalzenen Glyzerins dosiert, die beim Vorhandensein der zu testenden Substanz "in vitro" inkubiert wurden.

Die Adipozyten werden durch enzymatische Verdauung des subkutanen menschlichen Gewebes nach der sogenannten Rodbell-Technik (Rodbell M: "Metabolism of isolated fat cells", The journal of biological chemistry, vol. 239, n° 2, Seite 375 bis 380, 1964) befreit.

Die Pflanzenextrakte werden in einem definierten Medium aufgelöst, das 90 Minuten lang bei 37° C in Kontakt mit den Adipozyten gebracht wird.

Die Rate des ausgesalzenen Glyzerins wird durch Spektrophotometrie in einem Medium quantifiziert, das nach der de Carpéné C. und Koll.-Technik (J. Pharmacol., vol. 12, n° 2, Seite 219-224, 1981) an der Oberfläche schwimmt.

Die Rate des freigesetzten Glyzerins wird im Verhältnis zur Rate aller durch Turbidimetrie dosierten Lipide angegeben. Das Ergebnis wird in prozentualer Erhöhung im Verhältnis zum Kontrollmittel ohne Pflanzenextrakt angegeben.

Die Vergleichssubstanzen sind Theophyllin und Isoprenalin.

Die Ergebnisse der Testserien **V), VI) und VII)** werden in der folgenden Tabelle zusammengefasst:

| Pflanze | Extrakttyp | Tyrosinase | | Anti-Glykation (% I) | | B 16-Melanin | | | Lipolyse |
|---|---|---|---|---|---|---|---|---|---|
| | | C150 | CA50 | fs | fgc | Dosis | Index | Mel. Extra | % Steigerung |
| Clidemia hirta | wässriger E. | 0,31 | | 37 | 50 | | | | |

(fortgesetzt)

| Pflanze | Extrakttyp | Tyrosinase | | Anti-Glykation (% I) | | B 16-Melanin | | | Lipolyse |
|---|---|---|---|---|---|---|---|---|---|
| | | C150 | CA50 | fs | fgc | Dosis | Index | Mel. Extra | % Steigerung |
| Inga bourg. | wässriger E. | 0,060 | | 100 | 76 | 0,05 | 3,9 | 68 | |
| | Äthanol E. 50% | 0,070 | | | | 0,03 | 3,5 | 80 | |
| | Äthanol-E. | 0,010 | | | | 0,02 | 1,4 | 10 | |
| Sabicea cinerea | wässriger E. | | | 21 | 50 | | | | |
| | Äthanol E. 50% | 0,090 | | | | 0,01 | 1,6 | 49 | |
| | Äthanol-E. | 0,060 | | | | 0,03 | 1,8 | 10 | |
| Astroc. vulgere | wässriger E. | | | | | | | | |
| | Äthanol E. 50% | 0,63 | | | | | | | |
| | Äthanol-E. | 0,23 | | | | | | | |
| Siparuna guianensis | wässriger E. | | | | | | | | |
| | Methanol E.80% | | | | | | | | |
| | Äthanol-E. | | | | | | | | |
| Eperua falcata | wässriger E. | | | 97 | 46 | | | | |
| | Methanol E.80% | 0,38 | | | | | | | 30 |
| | Äthanol-E. | 0,080 | | | | 0,01 | 1,5 | 93 | |
| Byrsonima verb. | wässriger E. | 0,090 | | | | 0,02 | 1,7 | 72 | |
| | Methanol E.80% | 0,050 | | | 0,02 | 2,5 | 91 | | |
| | Äthanol-E. | 0,010 | | | 0,03 | 2,5 | 58 | | |
| Priva lappulacea | wässriger E. | 0,35 | | | | | | | |
| | Methanol E.80% | 0,13 | | | | | | | |
| | Äthanol-E. | 0,060 | | | | | | | |
| Goupia glabra | wässriger E. | | 0,037 | | | | | | |
| | Methanol E.80% | | | | | | | | |

(fortgesetzt)

| Pflanze | Extrakttyp | Tyrosinase | | Anti-Glykation (% I) | | B 16-Melanin | | | Lipolyse |
|---|---|---|---|---|---|---|---|---|---|
| | | C150 | CA50 | fs | fgc | Dosis | Index | Mel. Extra | % Steigerung |
| Coutoubea spicata | wässriger E. | 0,64 | | | | | | | |
| | Methanol E.80% | 0,39 | | | | | | | |
| | Äthanol-E. | 0,27 | | | | | | | |

In der oben stehenden Tabelle:

- C150: Konzentration aktiver Stoffe in Gewicht/Volumen, die eine 50 %ige Hemmung geben.
- CA50: Konzentration aktiver Stoffe in Gewicht/Volumen, die eine 50 %ige Aktivierung geben
- Dosis: Konzentration aktiver Stoffe, die im Gewicht/Volumen getestet wurden.
- Index (B16-Melanin): Proteinrate/Rate des intrazellularen Melanins.
- Mel. Extra: Rate des extrazellularen Melanins (in dem auf der Oberfläche schwimmenden Stoff ausgesalzen) in prozentualer Hemmung.

Bei der Auswertung der Ergebnisse der obigen Testtabellen haben die Erfinder verschiedene, bevorzugte Ausführungsarten der Erfindung ausgearbeitet.

Um eine kosmetische oder dermopharmazeutische Zusammensetzung zu erhalten, die ausser der anti-radikalartigen Aktivität auch eine verstärkte Anti-Elastase-Aktivität bietet, wurde das in die besagte Zusammensetzung aufgenommene aktive Mittel und das diese Eigenschaften übermittelt, auf vorteilhafte Weise durch mindestens einen Extrakt einer Pflanze gebildet, die aus der durch Clidemia hirta, Inga bourgoni, Sabicea cinerea, Astrocaryum vulgare, Siparuna guianensis, Eperua falcata, Byrsonima verbascifolia und Priva lappulacea gebildeten Gruppe gewählt.

Um eine kosmetische oder dermopharmazeutische Zusammensetzung zu erhalten, die ausser der anti-radikatartigen Aktivität auch eine verstärkte Anti-KollagenaseAktivität bietet, wird das aktive Mittel, das in die besagte Zusammensetzung angenommen wird und diese Eigenschaften übermittelt, auf vorteilhafte Weise durch einen Extrakt einer Pflanze gebildet, die aus der durch Clidemia hirta, Inga bourgoni, Sabicea cinerea, Astrocaryum vulgare, Siparuna guianensis, Eperua falcata, Byrsonima verbascifolia und Priva lappulacea gebildeten Gruppe gewählt.

Um eine kosmetische oder dermopharmazeutische Zusammensetzung zu erhalten, die ausser der anti-radikalartigen Aktivität auch eine verstärkte entpigmentierende Aktivität bietet, wird das aktive Mittel, das in die besagte Zusammensetzung aufgenommen wird und diese Eigenschaften übermittelt, auf vorteilhafte Weise durch mindestens einen Extrakt einer Pflanze gebildet, die aus der durch Clidemia hirta, Inga bourgoni, Sabicea cinerea, Eperua falcata, Byrsonima verbascifolia, Priva lappulacea, Astrocaryum vulgare und Coutoubea spicata gebildeten Gruppe gewählt.

Um eine kosmetische oder dermopharmazeutische Zusammensetzung zu erhalten, die ausser der anti-radikalartigen Aktivität auch eine wichtige Anti-UVB-Aktivität bietet, wird das aktive Mittel, das in die besagte Zusammensetzung aufgenommen wird und diese Eigenschaften übermittelt, auf vorteilhafte Weise durch mindestens einen Extrakt einer Pflanze gebildet, die aus der durch Clidemia hirta, Inga bourgoni, Sabicea cinerea, Astrocaryum vulgare, Siparuna guianensis, Eperua falcata, Byrsonima verbascifolia und Priva lappulacea gebildeten Gruppe gewählt.

Um eine kosmetische oder dermopharmazeutische Zusammensetzung zu erhalten, die ausser der anti-radikalartigen Aktivität auch eine wichtige Anti-UVA-Aktivität bietet, wird das aktive Mittel, das in die besagte Zusammensetzung aufgenommen wird und diese Eigenschaften übermittelt, auf vorteilhafte Weise durch mindestens einen Extrakt einer Pflanze gebildet, die aus der durch Clidemia hirta, Inga bourgoni, Sabicea cinerea, Astrocaryum vulgare, Siparuna guianensis, Eperua falcata, Byrsonima verbascifolia und Priva lappulacea gebildeten Gruppe gewählt.

Um eine kosmetische oder dermopharmazeutische Zusammensetzung zu erhalten, die ausser der anti-radikalartigen Aktivität auch eine Aktivität bietet, die die Katalase gegen UVA schützt, wird das aktive Mittel, das in die besagte Zusammensetzung aufgenommen wird und diese Eigenschaften übermittelt, auf vorteilhafte Weise, durch mindestens einen Extrakt einer Pflanze gebildet, die aus der durch Sabicea cinerea, Siparuna guianensis und Byrsonima verbascifolia gebildeten Gruppe gewählt.

Um eine kosmetische oder dermopharmazeutische Zusammensetzung zu erhalten, die ausser der anti-radikalartigen Aktivität auch eine hohe Anti-Glykations-Aktivität bietet, wird das aktive Mittel, das in die besagte Zusammensetzung aufgenommen wird und diese Eigenschaften übermittelt, auf vorteilhafte Weise, durch mindestens einen Extrakt einer Pflanze gebildet, die aus durch Clidemia hirta, Inga bourgoni, Sabicea cinerea und Eperua falcata gebildeten Gruppe gewählt.

Um eine kosmetische oder dermopharmazeutische Zusammensetzung zu erhalten, die ausser der anti-radikalartigen Aktivität auch eine wichtige pigmentierende Aktivität bietet, wird das aktive Mittel, das in die besagte Zusammensetzung aufgenommen wird und diese Eigenschaften übermittelt, auf vorteilhafte Weise durch einen Extrakt der Goupia glabra-Pflanze gebildet.

Um eine kosmetische oder dermopharmazeutische Zusammensetzung zu erhalten, die ausser der anti-radikalartigen Aktivität auch eine wesentliche Schlankheitsfördernde- (lipolytische) Aktivität bietet, wird das aktive Mittel, das in die besagte Zusammensetzung aufgenommen wird und diese Eigenschaften übermittelt, auf vorteilhafte Weise durch einen Extrakt der Eperua falcata-Pflanze gebildet.

Vorzugsweise wird das aktive Mittel jedoch durch einen Extrakt oder ein Gemisch aus Extrakten gebildet, der/das aus einer Pflanze oder mehreren Pflanzen gewonnen wird, der/das aus der durch Clidemia hirta, Inga bourgoni, Sabicea cinerea, Astrocaryum vulgare, Siparuna guianensis, Eperua falcata, Byrsonima verbascifolia und Priva lappulacea gebildeten Gruppe gewählt.

Die Zielsetzung der vorliegenden Erfindung betrifft ebenfalls eine kosmetische oder dermopharmazeutische Zusammensetzung für den lokalen, äusseren Gebrauch für die Haut, die Schleimhäute und/oder das Epithel- oder Körperanhanggebilde, dadurch gekennzeichnet, dass sie als aktiver Wirkstoff, der besonders eine anti-radikalartige Aktivität bietet, alleine oder mit mindestens einem anderen hinzugefügten Mittel, einen Extrakt einer Pflanze enthält, deren botanische Gattung zur Gruppe gehört, die durch die Gattungen Clidemia, Inga, Sabicea, Astrocaryum, Siparuna, Eperua, Byrsonima, Priva, Coutoubea und Goupia gebildet wird, vorzugsweise eine Pflanze, die in der durch Clidemia hirta, Inga bourgoni, Sabicea cinerea, Astrocaryum vulgare, Siparuna guianensis, Eperua falcata, Byrsonima verbascifolia, Priva lappulacea, Coutoubea spicata und Goupia glabra gebildeten Gruppe gewählt wird.

Je nach den anderen, zusätzlichen Eigenschaften im Verhältnis zur anti-radikalartigen Aktivität, die die kosmetische oder dermopharma-zeutische Zusammensetzung eventuell bieten muss, wird die Gruppe der Pflanzen unter denen, die vorab erwähnt wurden, die gewählt werden kann, verschiedene Vertreter nach den Aktivitäten jeder einzelnen unter ihnen enthalten, die aus den Tabellen der Ergebnisse der oben angegebenen Tests hervorgehen.

So kann nach einer ersten Variante der Ausführung nach der Erfindung die kosmetische oder dermopharmazeutische Zusammensetzung ein aktiver Wirkstoff mit Anti-UVA-, Anti-UVB- und Anti-Kollagenase-Aktivitäten enthalten, das durch mindestens ein Extrakt einer Pflanze gebildet wird, die aus der durch Clidemia hirta, Inga bourgoni, Sabicea cinerea, Astrocaryum vulgare, Siparuna guianensis, Eperua falcata, und Byrsonima verbascifolia gebildeten Gruppe gewählt wird.

Nach einer zweiten Variante der Ausführung nach der Erfindung kann die kosmetische oder dermopharmazeutische Zusammensetzung einen aktiven Wirkstoff mit Anti-UVA-, Anti-UVB-, Anti-Kollagenase- und Anti-Elastase-Aktivitäten enthalten, der durch mindestens einen Extrakt einer Pflanze gebildet wird, die aus der durch Clidemia hirta, Inga bourgoni, Sabicea cinerea, Astrocaryum vulgare, Siparuna guianensis, Eperua falcata und Byrsonima verbascifolia gebildeten Gruppe gewählt wird.

Nach einer dritten Variante der Ausführung nach der Erfindung kann die kosmetische oder dermopharmazeutische Zusammensetzung einen aktiven Wirkstoff mit Anti-UVA-, Anti-UVB-, Anti-Kollagenase- und Anti-Glykation-Aktivitäten enthalten, der durch mindestens einen Extrakt einer Pflanze gebildet wird, die aus der durch Clidemia hirta, Inga bourgoni, Sabicea cinerea, Eperua falcata gebildeten Gruppe gewählt wird.

Nach einer vierten Variante der Ausführung nach der Erfindung kann die kosmetische oder dermopharmazeutische Zusammensetzung einen aktiven Wirkstoff mit Anti-UVA-, Anti-UVB-, Anti-Kollagenase-, Anti-Elastase- und entpigmentierende Aktivitäten enthalten, der durch mindestens einen Extrakt einer Pflanze gebildet wird, die aus der durch Clidemia hirta, Inga bourgoni, Sabicea cinerea, Eperua falcata und Byrsonima verbascifolia gebildeten Gruppe gewählt wird.

Nach einer fünften Variante der Ausführung nach der Erfindung kann die kosmetische oder dermopharmazeutische Zusammensetzung einen aktiven Wirkstoff mit pigmentierender Aktivität enthalten, der durch einen Extrakt der Goupia glabra-Pflanze gebildet wird.

Nach einer sechsten Variante der Ausführung nach der Erfindung kann die kosmetische oder dermopharmazeutische Zusammensetzung einen aktiven Wirkstoff mit Schlankheitsfördernde-Aktivität enthalten, der durch einen Extrakt der Eperua falcata-Pflanze gebildet wird.

Auf vorteilhafte Weise enthält die kosmetische Zusammensetzung als aktiven Wirkstoff alleine oder mit anderen aktiven Wirkstoffen verbunden, zwischen 0,001 % und 20 % im Gewicht, vorzugsweise zwischen 0,1 % und 3 % im Gewicht eines Extrakts oder eines Gemischs aus Extrakten von einer Pflanze/n, die oben stehend definiert und gewonnen wurden.

Der Extrakt oder das Gemisch der Extrakte, der/das einen aktiven Wirkstoff bildet, kann in jeglicher galenischer, in der Kosmetik verwendbarer Form benutzt werden, besonders in einer galenischen Form, die aus den durch Öl-in-Wasser-Emulsionen, den Wasser-in-Öl-Emulsionen, Gesichtswassem, kosmetischer Milch, Gelen, Hydrogelen, Cremes, Pommaden, Seifen, Stäbchen, Sprühmittel, Haarwasser und Shampoos gewählt wird.

Ausserdem kann der Extrakt oder das Gemisch aus Extrakten zu einem oder mehreren kosmetischen Vektorlen hinzugefügt werden, besonders zu einem oder mehreren Vektor/en, der/die in der durch Liposome, Makrokapseln, Mikrokapseln, Nanokapseln, Makropartikeln, Mikropartikeln und Nano-partikeln gebildeten Gruppe gewählt wurden.

Als Beispiele, die nicht einschränkend für die praktische Durchführung nach der Erfindung sind, werden nachstehend verschiedene Erzeugnisse oder kosmetische Präparate beschrieben, die einen wie vorab beschriebenen Pflanzenextrakt enthalten.

Beispiel 1

Ein kosmetisches Erzeugnis in der Form einer Bleichcreme für die Haut kann zum Beispiel die folgende Gewichtszusammensetzung aufweisen:

| Fraktion A: | |
|---|---|
| Glyzerinstereat und Ceteareth-20 | 15,0 % |
| Paraffinöl | 3,0 % |
| Ascorbyl-Palmitat | 3,0 % |
| Dimethicon | 3,0 % |
| Cetylalkohol | 0,5 % |
| PEG-30 Glyzerol-Isostearat | 2,0 % |
| Fraktion B: | |
| Wasser | 72,2 % |
| Methylparaben | 0,2 % |
| Imidazolinidyl-Harnstoff | 0,3 % |
| Äthanolextrakt aus Inga bourgoni | 0,5 % |
| Fraktion C: | |
| Parfum | 0,3 % |

Der Vorgang für die Herstellung dieser Creme besteht darin die Bestandteile der Fraktion A unter Rühren bei 75° C schmelzen zu lassen, die Fraktion B bei 75° C vorzubereiten und dann die Fraktion B unter Turbinenrühren in die Fraktion A zu schütten, unter Planetenrühren abkühlen zu lassen und dann zur Fraktion C hinzuzufügen.

Beispiel 2

Ein kosmetisches Erzeugnis in der Form einer Anti-Flecken-Emulsion für die Hände, die kutane Pigmentflekken behandeln soll, kann zum Beispiel die nachstehend angegebene Gewichtszusammensetzung aufweisen.

Dieses Erzeugnis könnte auch nach der Erfindung ein multiaktives Erzeugnis bilden, besonders ein antiradikalartiges, Anti-Elastase und Anti-Kollagenase.

| Fraktion A: | |
|---|---|
| Glyzerin-Stearat und PEG 100-Stereat | 6,0 % |
| Oleinalkohol | 1,5 % |
| Glyzerin-Stearat | 2,0 % |
| Steareth-2 | 2,0 % |
| Karité-Butter | 3,0 % |
| Dimethicon | 4,0 % |
| Kapryl-Kaprin-Triglycerid | 8,0 % |
| Propylparaben | 0,1 % |
| Tocopherol-Azetat | 0,1 % |
| | |
| Fraktion B: | |
| Wasser | 60,8 % |

(fortgesetzt)

| Fraktion B: | |
|---|---|
| Elastab 388 (Laboratoires Serobiologiques) | 2,5 % |
| Extrakt von Byrsonima verbascifolia (80 % Methanol) | 1,5 % |
| Äthanol-Astrocaryum vulgare-Extrakt | 1,5 % |
| Propylen-Glykol | 5,0 % |
| | |
| Fraktion C: | |
| Polyacrylamid und Isoparaffin und Laureth 7 | 2,0 % |

Der Vorgang für die Herstellung dieser Emulsion besteht in der separaten Präparation der Fraktionen A und B bei 75° C und im Hinzufügen der Fraktion A in die Fraktion B unter Turbinenrühren bei 75° C, in der Abkühlung bei 50° C und danach im Hinzufügen der Fraktion C und schliesslich in der Abkühlung des endgültigen Gemischs auf Raumtemperatur.

Beispiel 3

Ein kosmetisches Erzeugnis in der Form einer Anti-Falten Tages- und einer multiaktiven Anti-Alterserscheinungs-Creme kann zum Beispiel die folgende Gewichtszusammensetzung aufweisen:

| Fraktion A: | |
|---|---|
| Glyzerin-Stearat | 14,0 % |
| Octyldodecanol | 16,0 % |
| Dibutyl-Adipat | 6,0 % |
| Ceteareth 12 | 1,5 % |
| Ceteareth 20 | 1,5 % |
| | |
| Fraktion B: | |
| Propylen-Glykol | 5,0 % |
| wässriger Extrakt aus Inga bourgoni | 3,25 % |
| Extrakt aus Sabicea cinerea (50 % Äthanol) | 1,0 % |
| Äthanolextrakt aus Eperua falcata | 0,75 % |
| Elastab 4112 (Laboratoires Serobiologiques) | 0,4 % |
| Wasser | 50,6 % |
| | |
| Fraktion C: | |
| Parfum | 0,3 % |

Der Vorgang für die Herstellung dieser Creme besteht in der separaten Präparation der Fraktionen A und B bei 80° C unter Rühren, dem Hinzufügen der Fraktion A in die Fraktion B unter Turbinenrühren, der Abkühlung des Gemischs auf 45° C, dann im Hinzufügen der Fraktion C und schliesslich das Zurückbringen des endgültigen Gemischs auf Raum-temperatur.

**Patentansprüche**

1. Verwendung eines Extrakts einer Pflanze deren botanische Gattung zur Gruppe gehört, die von den Clidemia-, Inga-, Sabicea-, Astrocaryum-, Siparuna-, Eperua-, Byrsonima-, Priva-, Coutoubea- und Goupia-Gattungen gebildet wird, für die Präparation eines kosmetischen oder dermopharmazeutischen Erzeugnisses für einen lokalen, äusseren Gebrauch für die Haut, die Schleimhäute und/oder das Epithel- oder Körperanhanggebilde, das besonders anti-radikalartige Aktivitäten aufweist.

**2.** Verwendung nach Anspruch 1, **dadurch gekennzeichnet, dass** der aktive Wirkstoff mindestens aus einem Extrakt einer Pflanze besteht, die aus der von Clidemia hirta, Inga bourgoni, Sabicea cinerea, Astrocaryum vulgare, Siparuna guianensis, Eperua falcata, Byrsonima verbascifolia, Priva lappulacea, Coutoubea spicata und Goupia glabra gebildeten Gruppe gewählt wurde.

**3.** Verwendung nach einer der Ansprüche 1 und 2, **dadurch gekennzeichnet, dass** die für die Präparation der Extrakte verwendeten Teile der Pflanzen die Wurzeln, Rinden, Blätter, beblätterten Stengel, Früchte, Körner und/oder die Blüten sind.

**4.** Verwendung nach irgendeinem der Ansprüche 1 bis 3, **dadurch gekennzeichnet, dass** das für die Durchführung der Extraktionen verwendete Lösemittel in der Gruppe gewählt wird, die durch Wasser, Alkohole, Ketone, Ester, Äthere, Polyole, chlorhaltige Lösemittel und Gemische aus mindestens zwei der vorab erwähnten Lösemittel gebildet wird.

**5.** Verwendung nach irgendeinem der Ansprüche 1 bis 3, **dadurch gekennzeichnet, dass** der Extrakt oder die Extrakte durch einen Extraktionsvorgang gewonnen wird/werden, der auf einer Wellenbestrahlung basiert, wie zum Beispiel Mikrowellen oder Ultraschall.

**6.** Verwendung nach irgendeinem der Ansprüche 1 bis 3, **dadurch gekennzeichnet, dass** das für die Durchführung der Extraktionen verwendete Lösemittel superkritisches $CO_2$ ist, das alleine oder in einem Gemisch mit einem Beilösemittel verwendet wird.

**7.** Verwendung nach irgendeinem der Ansprüche 1 bis 6, **dadurch gekennzeichnet, dass** das aktive Mittel, das ausser einer verstärkten Anti-Elastase-Aktivität durch mindestens einen Extrakt einer Pflanze gebildet wird, die aus der durch Clidemia hirta, Inga bourgoni, Sabicea cinerea, Astrocaryum vulgare, Siparuna guianensis, Eperua falcata, Byrsonima verbascifolia und Priva lappulacea gebildeten Gruppe gewählt wird.

**8.** Verwendung nach irgendeinem der Ansprüche 1 bis 6, **dadurch gekennzeichnet, dass** das aktive Mittel, das ausserdem eine verstärkte entpigmentierende Aktivität bietet, durch mindestens einen Extrakt einer Pflanze gebildet wird, die aus der durch Clidemia hirta, Inga bourgoni, Sabicea cinerea, Eperua falcata, Byrsonima verbascifolia, Priva lappulacea, Coutouba spicata und Astrocaryum vulgare gebildeten Gruppe gewählt wird.

**9.** Verwendung nach irgendeinem der Ansprüche 1 bis 6, **dadurch gekennzeichnet, dass** das aktive Mittel, das ausserdem eine verstärkte Anti-Kollagenase-Aktivität aufweist, aus mindestens einem Extrakt einer Pflanze gebildet wird, die aus der durch Clidemia hirta, Inga bourgoni, Sabicea cinerea, Astrocaryum vulgare, Siparuna guianensis, Eperua falcata, Byrsonima verbascifolia, Priva lappulacea gebildeten Gruppe gewählt wird.

**10.** Verwendung nach irgendeinem der Ansprüche 1 bis 6, **dadurch gekennzeichnet, dass** das aktive Mittel, das ausserdem einen wichtigen Anti-UVB-Effekt aufweist, aus mindestens einem Extrakt einer Pflanze gebildet wird, die aus der durch Clidemia hirta, Inga bourgoni, Sabicea cinerea, Astrocaryum vulgare, Siparuna guianensis, Eperua falcata, Byrsonima verbascifolia und Priva lappulacea gebildeten Gruppe gewählt wird.

**11.** Verwendung nach irgendeinem der Ansprüche 1 bis 6, **dadurch gekennzeichnet, dass** der aktive Wirkstoff, der ausserdem einen wichtigen Anti-UVA-Effekt aufweist, aus mindestens einem Extrakt einer Pflzanze gebildet wird, die aus der durch Clidemia hirta, Inga bourgoni, Sabicea cinerea, Astrocaryum vulgare, Siparuna guianensis, Eperua falcata, Byrsonima verbascifolia und Priva lappulacea gebildeten Gruppe gewählt wird.

**12.** Verwendung nach irgendeinem der Ansprüche 1 bis 6, **dadurch gekennzeichnet, dass** der aktive Wirkstoff, der ausserdem eine Schutzaktivität der Katalase gegen UVA aufweist, aus mindestens einem Extrakt einer Pflanze gebildet wird, die aus der durch Sabicea cinerea, Siparuna guianensis, Byrsonima verbascifolia gebildeten Gruppe gewählt wird.

**13.** Verwendung nach irgendeinem der Ansprüche 1 bis 6, **dadurch gekennzeichnet, dass** der aktive Wirkstoff, der ausserdem eine hohe Anti-Glykations-Aktivität aufweist, aus mindestens einem Extrakt einer Pflanze gebildet wird, die aus der durch Clidemia hirta, Inga bourgoni, Sabicea cinerea und Eperua falcata gebildeten Gruppe gewählt wird.

**14.** Verwendung nach irgendeinem der Ansprüche 1 bis 6, **dadurch gekennzeichnet, dass** der aktive Wirkstoff, der ausserdem eine verstärkte Schlankheitsfördernde-Aktivität aufweist, aus mindestens einem Extrakt der Eperua falcata-Pflanze besteht.

15. Verwendung nach irgendeinem der Ansprüche 1 bis 6, **dadurch gekennzeichnet, dass** der aktive Wirkstoff, der ausserdem eine wichtige pigmentierende Aktivität aufweist, aus mindestens einem Extrakt der Goupia glabra-Pflanze besteht.

16. Verwendung nach irgendeinem der Ansprüche 1 bis 13, **dadurch gekennzeichnet, dass** der aktive Wirkstoff aus einem Extrakt oder einem Gemisch aus Extrakten besteht, der/das aus einer oder aus mehreren Pflanzen besteht, die aus der durch Clidemia hirta, Inga bourgoni, Sabicea cinerea, Astrocaryum vulgare, Siparuna guianensis, Eperua falcata, Byrsonima verbascifolia und Priva lappulacea gebildeten Gruppe gewählt wirdl werden.

17. Kosmetische oder dermopharmazeutische Zusammensetzung für lokalen, äusseren Gebrauch für die Haut, die Schleimhäute und/oder das Epithel- oder Körperanhanggebilde, **dadurch gekennzeichnet, dass** sie als aktiven Wirkstoff, der besonders eine anti-radikalartige Aktivität aufweist, alleine oder mit mindestens einem anderen Wirkstoff assoziiert, mindestens einen Extrakt einer Pflanze enthält, deren botanische Gattung bzw. Art zur der durch die Clidemia-, Sabicea-, Astrocaryum-, Siparuna-, Eperua-, Priva-, Coutoubea- und Goupia- Gattungen und die Inga bourgoni, Inga pezizifera, Inga alata, Inga alba, Inga capitata, Inga meissneriana, Byrsonima chrysophylla und Byrsonima coriacea Arten gebildeten Gruppe gehört.

18. Kosmetische oder dermopharmazeutische Zusammensetzung nach dem Anspruch 17, **dadurch gekennzeichnet, dass** die Pflanze in der durch Clidemia hirta, Inga bourgoni, Sabicea cinerea, Astrocaryum vulgare, Siparuna guianensis, Eperua falcata, Byrsonima chrysophylla, Byrsonima coriacea, Priva lappulacea, Coutoubea spicata und Goupia glabra gebildeten Gruppe gewählt wird.

19. Kosmetische oder dermopharmazeutische Zusammensetzung nach einem der Ansprüche 17 und 18, **dadurch gekennzeichnet, dass** sie einen aktiven Wirkstoff mit Anti-UVA-, Anti-UVB- und Anti-Kollagenase-Aktivitäten enthält, das aus mindestens einem Extrakt einer Pflanze gebildet ist, die in der durch Clidemia hirta, Inga bourgoni, Sabicea cinerea, Astrocaryum vulgare, Siparuna guianensis, Eperua falcata, Byrsonima chrysophylla und Byrsonima coriacea gebildeten Gruppe gewählt wird.

20. Kosmetische oder dermopharmazeutische Zusammensetzung nach einem der Ansprüche 17 bis 19, **dadurch gekennzeichnet, dass** sie einen aktiven Wirkstoff mit Anti-UVA-, Anti-UVB-, Anti-Kollagenase und Anti-Elastase-Aktivitäten enthält, dass er aus mindestens einem Extrakt einer Pflanze gebildet ist, die in der durch Clidemia hirta, Inga bourgoni, Sabicea cinerea, Astrocaryum vulgare, Siparuna guianensis, Eperua falcata, Byrsonima chrysophylla und Byrsonima coriacea gebildeten Gruppe gewählt wird.

21. Kosmetische oder dermopharmazeutische Zusammensetzung nach irgendeinem der Ansprüche 17 bis 19, **dadurch gekennzeichnet, dass** sie einen aktiven Wirkstoff mit Anti-UVA-, Anti-UVB-, Anti-Kollagenase und Anti-Glykation-Aktivitäten enthält, der aus mindestens einem Extrakt einer Pflanze gebildet ist, die in der durch Clidemia hirta, Inga bourgoni, Sabicea cinerea und Eperua falcata gebildeten Gruppe gewählt wird.

22. Kosmetische oder dermopharmazeutische Zusammensetzung nach irgendeinem der Ansprüche 17 bis 20, **dadurch gekennzeichnet, dass** sie einen aktiven Wirkstoff mit Anti-UVA-, Anti-UVB-, Anti-Kollagenase, Anti-Elastase- und entpigmentierenden Aktivitäten enthält, der aus mindestens einem Extrakt einer Pflanze gebildet ist, die in der durch Clidemia hirta, Inga bourgoni, Sabicea cinerea, Eperua falcata, Byrsonima chrysophylla und Byrsonima coriacea gebildeten Gruppe gewählt wird.

23. Kosmetische oder dermopharmazeutische Zusammensetzung nach einem der Ansprüche 17 und 18, **dadurch gekennzeichnet, dass** sie einen aktiven Wirkstoff mit einer pigmentierenden Aktivität enthält, der durch einen Extrakt einer Goupia glabra-Pflanze gebildet ist.

24. Kosmetische oder dermopharmazeutische Zusammensetzung nach einem der Ansprüche 17 und 18, **dadurch gekennzeichnet, dass** sie einen aktiven Wirkstoff mit Schlankheitsfördernde-Aktivität enthält, der durch einen Extrakt der Eperua falcata-Pfianze gebildet ist.

25. Kosmetische oder dermopharmazeutische Zusammensetzung nach irgendeinem der Ansprüche 17 bis 24, **dadurch gekennzeichnet, dass** sie als aktiven Wirkstoff alleine oder mit anderen aktiven Wirkstoffen verbunden

zwischen 0,001 % und 20 % im Gewicht, vorzugsweise zwischen 0,1 % und 3 % im Gewicht einen Extrakt oder ein Gemisch aus Extrakten aus Pflanzen enthält, die in der durch Clidemia hirta, Inga bourgoni, Sabicea cinerea, Astrocaryum vulgare, Siparuna guianensis, Eperua falcata, Byrsonima chrysophylla, Byrsonima coriacea, Priva lappulacea, Coutoubea spicata und Goupia glabra gebildeten Gruppe gewählt werden.

26. Kosmetische oder dermopharmazeutische Zusammensetzung nach irgendeinem der Ansprüche 17 bis 25, **dadurch gekennzeichnet, dass** der Extrakt oder das Gemisch aus Extrakten einen aktiven Wirkstoff bildet/n, der in galenischer Form, die in der Kosmetik verwendbar ist, benutzt wird, besonders in galenischer Form, die in der durch Öl-in-Wasser-Emulsionen, Wasser-in-Öl-Emulsionen, Gesichtswasser, Milch, Gelen, Hydrogelen, Cremes, Pommaden, Seifen, Stäbchen, Sprühmittel, Haarwasser und Shampoos gebildeten Gruppe gewählt wurden.

27. Kosmetische oder dermopharmazeutische Zusammensetzung nach irgendeinem der Ansprüche 17 bis 26, **dadurch gekennzeichnet, dass** der Extrakt oder das Gemisch aus Extrakten in einen oder mehrere kosmetische Vektore hinzugefügt wird, besonders in einen oder mehrere Vektore, der/die in der durch Liposome, Makrokapseln, Mikrokapseln, Nanokapseln, Makropartikeln, Mikropartikeln und Nanopartikeln gebildeten Gruppe gewählt werden.

## Claims

1. Use of an extract of a plant of a botanical genus belonging to the group formed by Clidemia, Inga, Sabicea, Astrocaryum, Siparuna, Eperua, Byrsonima, Priva, Coutoubea and Goupia for the preparation of a cosmetic or dermopharmaceutical product for topical, external use for the skin, mucous membrane and/or epithelium or body appendages, in particular with an anti-radical action.

2. Use according to claim 1, **characterised in that** the active ingredient comprises at least one extract of a plant selected from the group formed by Clidemia hirta, Inga bourgoni, Sabicea cinerea, Astrocaryum vulgare, Siparuna guianensis, Eperua falcata, Byrsonima verbascifolia, Priva lappulacea, Coutoubea spicata and Goupia glabra.

3. Use according to claim 1 or 2, **characterised in that** the parts of the plants used to prepare the extracts are the roots, cortices, leaves, foliated stalks, fruit, grains and/or blossoms.

4. Use according to any of claims 1 to 3, **characterised in that** the solvent used to carry out the extractions is selected from the group formed by water, alcohols, ketones, esters, ethers, polyhydric alcohols, chlorine-containing solvents and mixtures of at least two of the afore-mentioned solvents.

5. Use according to any of claims 1 to 3, **characterised in that** the extract or extracts is/are obtained by an extraction process based on wave irradiation, for example microwaves or ultrasound.

6. Use according to any of claims 1 to 3, **characterised in that** the solvent used to carry out the extractions is supercritical $CO_2$, alone or mixed with a secondary solvent.

7. Use according to any of claims 1 to 6, **characterised in that** the active ingredient, which apart from an increased anti-elastase action, is formed by at least one extract of a plant selected from the group formed by Clidernia hirta, Inga bourgoni, Sabicea cinerea, Astrocaryum vulgare, Siparuna guianensis, Eperua falcata, Byrsonima verbascifolia and Priva lappulacea.

8. Use according to any of claims 1 to 6, **characterised in that** the active ingredient, which also gives an increased de-pigmenting action, is formed by at least one extract of a plant selected from the group formed by Clidemia hirta, Inga bourgoni, Sabicea cinerea, Eperua falcata, Byrsonima verbascifolia, Priva lappulacea, Coutouba spicata and Astrocaryum vulgare.

9. Use according to any of claims 1 to 6, **characterised in that** the active ingredient, which also has an increased anti-collagenase action, is formed from at least one extract of a plant selected from the group formed by Clidemia hirta, Inga bourgoni, Sabicea cinerea, Astrocaryum vulgare, Siparuna guianensis, Eperua falcata, Byrsonima verbascifolia and Priva lappulacea.

10. Use according to any of claims 1 to 6, **characterised in that** the active ingredient, which also has an important anti-UVB effect, is formed from at least one extract of a plant selected from the group formed by Clidemia hirta, Inga bourgoni, Sabicea cinerea, Astrocaryum vulgare, Siparuna guianensis, Eperua falcata, Byrsonima verbascifolia and Priva lappulacea.

11. Use according to any of claims 1 to 6, **characterised in that** the active ingredient, which also has an important anti-UVA effect, is formed from at least one extract of a plant selected from the group formed by Clidemia hirta, Inga bourgoni, Sabicea cinerea, Astrocaryum vulgare, Siparuna guianensis, Eperua falcata, Byrsonima verbascifolia and Priva lappulacea.

12. Use according to any of claims 1 to 6, **characterised in that** the active ingredient, which also has an action protecting catalase from UVA, is formed from at least one extract of a plant selected from the group formed by Sabicea cinerea, Siparuna guianensis and Byrsonima verbascifolia.

13. Use according to any of claims 1 to 6, **characterised in that** the active ingredient, which also has a strong anti-glycation action, is formed from at least one extract of a plant selected from the group formed by Clidemia hirta, Inga bourgoni, Sabicea cinerea and Eperua falcata.

14. Use according to any of claims 1 to 6, **characterised in that** the active ingredient, which also has an increased slimming action, comprises at least one extract of the Eperua falcata plant.

15. Use according to any of claims 1 to 6, **characterised in that** the active ingredient, which also has an important pigmenting action, comprises at least one extract from the Goupia glabra plant.

16. Use according to any of claims 1 to 13, **characterised in that** the active ingredient comprises an extract or a mixture of extracts comprising one or more plants selected from the group formed by Clidemia hirta, Inga bourgoni, Sabicea cinerea, Astrocaryum vulgare, Siparuna guianensis, Eperua falcata, Byrsonima verbascifolia and Priva lappulacea.

17. A cosmetic or dermo-pharmaceutical composition for topical, external use for the skin, mucous membrane and/or epithelium or body appendages, **characterised in that** it contains as the active ingredient, particularly with an anti-radical-type action, alone or combined with at least one other active ingredient, at least one extract of a plant of a botanical genus and species belonging to the group formed by the Clidemia, Sabicea, Astrocaryum, Siparuna, Eperua, Priva, Coutoubea and Goupia genera and the Inga bourgoni, Inga pezizifera, Inga alata, Inga alba, Inga capitata, Inga meissneriana, Byrsonima chrysophylla and Byrsonima coriacea species.

18. A cosmetic or dermo-pharmaceutical composition according to claim 17, **characterised in that** the plant is selected from the group formed by Clidemia hirta, Inga bourgoni, Sabicea cinerea, Astrocaryum vulgare, Siparuna guianensis, Eperua falcata, Byrsonima chrysophylla, Byrsonima coriacea, Priva lappulacea, Coutoubea spicata and Goupia glabra.

19. A cosmetic or dermo-pharmaceutical composition according to claim 17 or 18, **characterised in that** it contains an active ingredient with an anti-UVA, anti-UVB and anti-collagenase action formed from at least one extract of a plant selected from the group formed by Clidemia hirta, Inga bourgoni, Sabicea cinerea, Astrocaryum vulgare, Siparuna guianensis, Eperua falcata, Byrsonima chrysophylla and Byrsonima coriacea.

20. A cosmetic or dermo-pharmaceutical composition according to any of claims 17 to 19, **characterised in that** it contains an active ingredient with an anti-UVA, anti-UVB, anti-collagenase and anti-elastase action, and that the active ingredient is formed from at least one extract of a plant selected from the group formed by Clidemia hirta, Inga bourgoni, Sabicea cinerea, Astrocaryum vulgare, Siparuna guianensis, Eperua falcata, Byrsonima chrysophylla and Byrsonima coriacea.

21. A cosmetic or dermo-pharmaceutical composition according to any of claims 17 to 19, **characterised in that** it contains an active ingredient with an anti-UVA, anti-UVB, anti-collagenase and anti-glycation action, which is formed from at least one extract of a plant selected from the group formed by Clidemia hirta, Inga bourgoni, Sabicea cinerea and Eperua falcata.

22. A cosmetic or dermo-pharmaceutical composition according to any of claims 17 to 20, **characterised in that** it contains an active ingredient with an anti-UVA, anti-UVB, anti-collagenase, anti-elastase and de-pigmenting action, which is formed from at least one extract of a plant selected from the group formed by Clidemia hirta, Inga bourgoni, Sabicea cinerea, Eperua falcata, Byrsonima chrysophylla and Byrsonima coriacea.

23. A cosmetic or dermo-pharmaceutical composition according to claim 17 or 18, **characterised in that** it contains an active ingredient with a pigmenting action, formed by an extract from a Goupia glabra plant.

24. A cosmetic or dermo-pharmaceutical composition according to claim 17 or 18, **characterised in that** it contains an active ingredient with a slimming action, formed by an extract from the Eperua falcata plant.

25. A cosmetic or dermo-pharmaceutical composition according to any of claims 17 to 24, **characterised in that** it contains as the active ingredient, alone or combined with other active ingredients, between 0.001% and 20% by weight, preferably between 0.1% and 3% by weight of an extract or a mixture of extracts from plants selected from the group formed by Clidemia hirta, Inga bourgoni, Sabicea cinerea, Astrocaryum vulgare, Siparuna guianensis, Eperua falcata, Byrsonima chrysophylla, Byrsonima coriacea, Priva lappulacea, Coutoubea spicata and Goupia glabra.

26. A cosmetic or dermo-pharmaceutical composition according to any of claims 17 to 25, **characterised in that** the extract or mixture of extracts form an active ingredient which is used in galenic form possible in cosmetics, particularly in a galenic form chosen in the group formed by oil in water emulsions, water in oil emulsions, face lotions, cosmetic milk, gels, hydrogels, cremes, pomades, soaps, small sticks, spraying materials, hair lotion and shampoos.

27. A cosmetic or dermo-pharmaceutical composition according to any of claims 17 to 26, **characterised in that** the extract or mixture of extracts is added to one or more cosmetic vectors, particularly one or more vectors selected from the group formed by liposomes, macrocapsules, microcapsules, nanocapsules, macroparticles, microparticles and nano-particles.

**Revendications**

1. Utilisation d'un extrait d'une plante dont le genre botanique appartient au groupe formé par les genres Clidemia, Inga, Sabicea, Astrocaryum, Siparuna, Eperua, Byrsonima, Priva, Coutoubea et Goupia, pour la préparation d'un produit cosmétique ou dermopharmaceutique à usage topique externe pour la peau, les muqueuses et/ou les phanères, présentant particulièrement des activités anti-radicalaires.

2. Utilisation selon la revendication 1, **caractérisée en ce que** l'agent actif est constitué par au moins un extrait d'une plante choisie dans le groupe formé par Clidemia hirta, Inga bourgoni, Sabicea cinerea, Astrocaryum vulgare, Siparuna guianensis, Eperua falcata, Byrsonima verbascifolia, Priva lappulacea, Coutoubea spicata et Goupia glabra.

3. Utilisation selon l'une quelconque des revendications 1 et 2, **caractérisée en ce que** les parties de plantes utilisées pour les préparations des extraits sont les racines, les écorces, les feuilles et tiges feuillées, les fruits, les graines et/ou les fleurs.

4. Utilisation selon l'une quelconque des revendications 1 à 3, **caractérisée en ce que** le solvant mis en oeuvre pour la réalisation des extractions est choisi dans le groupe formé par l'eau, les alcools, les cétones, les esters, les éthers, les polyols, les solvants chlorés et les mélanges d'au moins deux des solvants précités.

5. Utilisation selon l'une quelconque des revendications 1 à 3, **caractérisée en ce que** l'extrait ou les extraits sont obtenus au moyen d'un procédé d'extraction basé sur un rayonnement ondulatoire, tel que les micro-ondes ou les ultrasons.

6. Utilisation selon l'une quelconque des revendications 1 à 3, **caractérisée en ce que** le solvant mis en oeuvre pour la réalisation des extractions est le $CO_2$ supercritique, seul ou en mélange avec un co-solvant.

7. Utilisation selon l'une quelconque des revendications 1 à 6, **caractérisée en ce que** l'agent actif, présentant en plus une activité anti-élastase renforcée, est constitué par au moins un extrait de plante choisie dans le groupe formé par Clidemia hirta, Inga bourgoni, Sabicea cinerea, Astrocaryum vulgare, Siparuna guianensis, Eperua falcata, Byrsonima verbascifolia et Priva lappulacea.

8. Utilisation selon l'une quelconque des revendications 1 à 6, **caractérisée en ce que** l'agent actif, présentant en plus une activité dépigmentante renforcée, est constitué par au moins un extrait de plante choisie dans le groupe formé par Clidemia hirta, Inga bourgoni, Sabicea cinerea, Eperua falcata, Byrsonima verbascifolia, Priva lappulacea, Coutoubea spicata et Astrocaryum vulgare.

9. Utilisation selon l'une quelconque des revendications 1 à 6, **caractérisée en ce que** l'agent actif, présentant en plus une activité anti-collagénase renforcée, est constitué par au moins un extrait de plante choisie dans le groupe formé par Clidemia hirta, Inga bourgoni, Sabicea cinerea, Astrocaryum vulgare, Siparuna guianensis, Eperua falcata, Byrsonima verbascifolia et Priva lappulacea.

10. Utilisation selon l'une quelconque des revendications 1 à 6, **caractérisée en ce que** l'agent actif, présentant en plus un effet anti-UVB important, est constitué par au moins un extrait de plante choisie dans le groupe formé par Clidemia hirta, Inga bourgoni, Sabicea cinerea, Astrocaryum vulgare, Siparuna guianensis, Eperua falcata, Byrsonima verbascifolia et Priva lappulacea.

11. Utilisation selon l'une quelconque des revendications 1 à 6, **caractérisée en ce que** l'agent actif, présentant en plus un effet anti-UVA important, est constitué par au moins un extrait de plante choisie dans le groupe formé par Clidemia hirta, Inga bourgoni, Sabicea cinerea, Astrocaryum vulgare, Siparuna guianensis, Eperua falcata, Byrsonima verbascifolia et Priva lappulacea.

12. Utilisation selon l'une quelconque des revendications 1 à 6, **caractérisée en ce que** l'agent actif, présentant en plus une activité protectrice de la catalase vis-à-vis des UVA, est constitué par au moins un extrait de plante choisie dans le groupe formé par Sabicea cinerea, Siparuna guianensis, Byrsonyma verbascifolia.

13. Utilisation selon l'une quelconque des revendications 1 à 6, **caractérisée en ce que** l'agent actif, présentant en plus une activité anti-glycation élevée, est constitué par au moins un extrait de plante choisie dans le groupe formé par Clidemia hirta, Inga bourgoni, Sabicea cinerea et Eperua falcata.

14. Utilisation selon l'une quelconque des revendications 1 à 6, **caractérisée en ce que** l'agent actif, présentant en plus une activité amincissante prononcée, est constitué par au moins un extrait de la plante Eperua falcata.

15. Utilisation selon l'une quelconque des revendications 1 à 6, **caractérisée en ce que** l'agent actif, présentant en plus une activité pigmentante importante, est constitué par au moins un extrait de la plante Goupia glabra.

16. Utilisation selon l'une quelconque des revendications 1 à 13, **caractérisée en ce que** l'agent actif est constitué par un extrait ou un mélange d'extraits obtenus à partir d'une plante ou de plusieurs plantes choisies dans le groupe formé par Clidemia hirta, Inga bourgoni, Sabicea cinerea, Astrocaryum vulgare, Siparuna guianensis, Eperua falcata, Byrsonima verbascifolia et Priva lappulacea.

17. Composition cosmétique ou dermopharmaceutique à usage topique externe pour la peau, les muqueuses et/ou les phanères **caractérisée en ce qu'**elle comporte à titre d'agent actif, présentant notamment une activité anti-radicalaire, seul ou associé à au moins un autre agent, au moins un extrait d'une plante dont le genre botanique appartient au groupe formé par les genres Clidemia, Sabicea, Astrocaryum, Siparuna, Eperua, Priva, Coutoubea et Goupia et les espèces Inga bourgoni, Inga pezizifera, Inga alata, Inga alba, Inga capitata, Inga meissneriana, Byrsonima chrysophylla et Byrsonima coriacea.

18. Composition cosmétique ou dermopharmaceutique selon la revendication 17, **caractérisée en ce que** la plante est choisie dans le groupe formé par Clidemia hirta, Inga bourgoni, Sabicea cinerea, Astrocaryum vulgare, Siparuna guianensis, Eperua falcata, Byrsonima chrysophylla, Byrsonima coriacea, Priva lappulacea, Coutoubea spicata et Goupia glabra.

19. Composition cosmétique ou dermopharmaceutique selon l'une quelconque des revendications 17 et 18, **caractérisée en ce qu'**elle comporte un agent actif à activités anti-UVA, anti-UVB et anti-collagénase, constitué par au moins un extrait de plante choisie dans le groupe formé par Clidemia hirta, Inga bourgoni, Sabicea cinerea, Astrocaryum vulgare, Siparuna guianensis, Eperua falcata, Byrsonima chrysophylla et Byrsonima coriacea.

**20.** Composition cosmétique ou dermopharmaceutique selon l'une quelconque des revendications 17 à 19, **caractérisée en ce qu'**elle comporte un agent actif à activités anti-UVA, anti-UVB, anti-collagénase et anti-élastase, constitué par au moins un extrait de plante choisie dans le groupe formé par Clidemia hirta, Inga bourgoni, Sabicea cinerea, Astrocaryum vulgare, Siparuna guianensis, Eperua falcata, Byrsonima chrysophylla et Byrsonima coriacea.

**21.** Composition cosmétique ou dermopharmaceutique selon l'une quelconque des revendications 17 à 19, **caractérisée en ce qu'**elle comporte un agent actif à activités anti-UVA, anti-UVB, anti-collagénase et anti-glycation, constitué par au moins un extrait de plante choisie dans le groupe formé par Clidemia hirta, Inga bourgoni, Sabicea cinerea et Eperua falcata.

**22.** Composition cosmétique ou dermopharmaceutique selon l'une quelconque des revendications 17 à 20, **caractérisée en ce qu'**elle comporte un agent actif à activités anti-UVA, anti-UVB, anti-collagénase, anti-élastase et dépigmentante, constitué par au moins un extrait de plante choisie dans le groupe formé par Clidemia hirta, Inga bourgoni, Sabicea cinerea, Eperua falcata, Byrsonima chrysophylla et Byrsonima coriacea.

**23.** Composition cosmétique ou dermopharmaceutique selon l'une quelconque des revendications 17 et 18, **caractérisée en ce qu'**elle comporte un agent actif à activité pigmentante constitué par un extrait de la plante Goupia glabra.

**24.** Composition cosmétique ou dermopharmaceutique selon l'une quelconque des revendications 17 et 18, **caractérisée en ce qu'**elle comporte un agent actif à activité amincissante constitué par un extrait de la plante Eperua falcata.

**25.** Composition cosmétique ou dermopharmaceutique selon l'une quelconque des revendications 17 à 24, **caractérisée en ce qu'**elle contient, en tant qu'agent actif, unique ou associé à d'autres agents actifs, entre 0,001 % et 20 % en poids, préférentiellement entre 0,1 % et 3 % en poids, d'un extrait ou d'un mélange d'extraits de plante (s) choisie(s) dans le groupe formé par Clidemia hirta, Inga bourgoni, Sabicea cinerea, Astrocaryum vulgare, Siparuna guianensis, Eperua falcata, Byrsonima chrysophylla, Byrsonima coriacea, Priva lappulacea, Coutoubea spicata et Goupia glabra.

**26.** Composition cosmétique ou dermopharmaceutique selon l'une quelconque des revendications 17 à 25, **caractérisée en ce que** l'extrait ou le mélange d'extraits forment un agent actif qui peut être employé sous une forme galénique utilisable en cosmétique, notamment sous une forme galénique choisie dans le groupe formé par les émulsions huile dans eau, les émulsions eau dans huile, les lotions pour le visage, les laits, les gels, les hydrogels, les crèmes, les pommades, les savons, les bâtonnets, les produits à pulvériser, les lotions capillaires et les shampooings.

**27.** Composition cosmétique ou dermopharmaceutique selon l'une quelconque des revendications 17 à 26, **caractérisée en ce que** l'extrait ou le mélange d'extraits est incorporé dans un ou plusieurs vecteurs cosmétiques, notamment un ou plusieurs vecteurs choisis dans le groupe formé par les liposomes, les macrocapsules, les microcapsules, les nanocapsules, les marcroparticules, les microparticules et les nanoparticules.